# EUROPEAN PATENT APPLICATION

(11) **EP 3 901 247 A1**
(43) Date of publication of application: **27.10.2021**
(21) Application number: 19897672.2
(22) Date of filing: 15.05.2019
(51) Int. Cl.: C12N 1/13, C12N 15/113, C12R 1/89

(54) **INDUCIBLE CRGPDH3 PROMOTER OF CHLAMYDOMONAS REINHARDTII AND THE USE THEREOF FOR THE EXPRESSION OF RECOMBINANT PROTEINS**

(30) Priority: 17.12.2018 MX 2018015859
(71) Applicant: Centro de Investigación Científica de Yucatán, A.C, 97205 Mérida, Yucatán (MX)
(72) Inventor: HERRERA VALENCIA, Virginia Aurora, Mérida, Yucatán, 97205 (MX); PERAZA ECHEVERRÍA, Santy, Mérida, Yucatán, 97205 (MX); BELTRÁN AGUILAR, Anayeli Guadalupe, Mérida, Yucatán, 97205 (MX)
(74) Representative: Elzaburu S.L.P.
(86) International application number: PCT/MX2019/000056
(87) International publication number: WO 2020/130772

(57) **Abstract**

The invention relates to an inducible nuclear promoter isolated from the freshwater green microalgae *Chlamydomonas reinhardtii* (*CrGPDH3* promoter), which was originally used to promote the expression of the CrGPDH3 gene which codes for the enzyme glycerol 3-phosphate dehydrogenase. The invention comprises a core promoter, a region of inducibility and chimeric promoters including said core promoter and the region of inducibility. The advantages of the present invention include the fact that it is a nuclear promoter, regulated by inducers which are easy to obtain and to apply, with low toxicity and low cost, such as NaCl. This promoter represents an alternative for the regulation of gene expression in photosynthetic organisms and it is useful for heterologous gene expression for the purpose of basic research or for biotechnological purposes, such as the production of recombinant proteins.

## Description

### TECHNICAL FIELD OF INVENTION

This invention belongs to the field of biotechnology, specifically to molecular biotechnology since it refers to a DNA sequence that belongs to the inducible promoter of *CrGPDH3* gene, here termed *CrGPDH3* promoter, isolated from the green microalga *Chlamydomonas reinhardtii,* and useful for the regulation of the genetic expression and the controlled expression of recombinant proteins in microalgae and other photosynthetic organisms.

### BACKGROUND OF THE INVENTION

Recombinant protein market ascends to 100 thousand million dollars annually (Walsh, 2010). In 2012, recombinant protein market listed in approximately $63.6 thousand million dollars, only in the United States, with a growth rate of 18.3% in relation to the year 2011 (Aggarwal 2014), thus concluding that the demand for these products will increase in the following years. Nowadays, recombinant proteins are a part of our daily lives and they will become indispensable products in various sectors of industry, such as pharmaceutical, food, energy and textile, traditional systems for the expression of recombinant proteins are the harvesting of bacteria (Rosano and Ceccarelli 2014), yeast (Celik and Calik 2012), insect cells (Drugmand *et al.* 2012) and mammal cells (Bandaranayake and Almo 2014). On the other hand, the harvesting of plant cells and transgenic plants are also being used for recombinant protein production since they offer advantages such as an easier harvest and management, lower costs and production time, and lower contamination risks with virus and prions (Sack *et al.* 2015). Recombinant protein market is growing (Sanchez-Garcia *et al.* 2016) and various productions systems must be exploited to fulfill the product demand and for this product to be safely and economically generated. The use of green microalgae as production system offers additional advantages, since it can provide higher yields in less time and lower productions costs than other systems, crops on a larger scale and environment-friendly, in addition to also being generally regarded as safe organisms (GRAS for its English acronym of 'generally regarded as safe') (Corchero *et al.* 2013). Microalgae are a part of a diverse group of photosynthetic microorganisms and they play an important role in the fixation of carbon and evolution of oxygen. These organisms have captured the attention for their biotechnological and industrial applications (Specht *et al.* 2010). Unicellular green microalga *Chlamydomonas reinhardtii* is an organism that has been used as a study model for understanding biological processes such as the metabolism of fatty acids and the metabolism of hydrogen, phototaxis, circadian cycle, the cycle of chloroplast, etc. (Scranton *et al.,* 2015). C. *reinhardtii* has been considered as an attractive system for recombinant protein production since it is easy to culture, it has relatively quick growth rate, it is feasible of genetic manipulation (the nuclear, chloroplast and mitochondria genomes have been fully sequenced), it is considered an innocuous microorganism (GRAS) and has a great capacity to accumulate biomass (Ramos-Martinez *et al.* 2017).

In terms of recombinant protein production cost, an example is the recombinant protein from amyloid bovine milk A (MAA) in the chloroplast of *C. reinhardtii,* where production cost could be significantly battered in relation to the sales price of commercial bovine colostrum (Gimpel *et al.* 2014), this allows us to acknowledge the use of microalgae as an excellent alternative for the recombinant protein production with a satisfactory cost/benefit ratio regarding other expression systems.

Today, there is an interest in transforming *C. reinhardtii* nucleus to express recombinant proteins, since this way there is the machinery to give direction of signal peptide, proteins secretion and to make complex post-translational processes as in the case of glycosylation (given that more than 70% of therapeutic proteins are glycosylated) (Scranton *et al.* 2015). Nevertheless, expressing recombinant proteins in the nucleus of *C. reinhardtii* has been more difficult than expressing them in the chloroplast, showing low accumulation of these, as well as effects of gene silencing. A key point to improving protein expression in the nucleus of *C. reinhardtii* is the search of promoters that can drive a high gene expression without causing toxic effects to the cell. Regarding *C. reinhardtii,* there is an expression system that contains a constitutive promoter (*HSP70A*/*RBCS2*) available in the market and distributed by the company Thermo Fisher Scientific®, mainly used for research purposes. Furthermore, although a few inducible promoters have been studied, as far as we know there are not strong nuclear inducible promoters for high expression of proteins in this microalga currently in the market.

An advantage of the use of inducible promoters to drive the expression of recombinant proteins in the nucleus of *C. reinhardtii,* is the possibility of controlling its temporary expression avoiding possible negative effects in the metabolism that affect cellular multiplication and the accumulation of biomass and thus of the recombinant protein. Regarding the state of the art, the inducible promoters that have been studied in this microalga have been very few. For example, NIT1 (Ohresser *et al.* 1997) which is an inducible promoter by ammonia deficiency in the culture media (TAP). Even though ammonia is an inductor that in small amounts is not toxic for the cell, it shows the disadvantage of the need of 24 hours for it to be induced and its expression levels are lower than those seen in the endogenous gene.

Promoter CA1 is induced by low concentrations of CO₂, nevertheless its disadvantages are that the maximum protein expression levels are reached after 48 hours of induction and the quantification of the reporter-protein was low with respect to its control (Villand *et al.* 1997).

For the FEA1 promoter induced by iron deprivation in the medium (TAP) reported by Allen et al. (2007), even though the expression levels that were obtained with this promoter were promising, it took 96 hours of induction to obtain those levels, which in terms of time and production is significantly limiting.

Promoter HSP70A is induced by thermal shock at a temperature of 40 ºC for an hour (Schroda *et al.* 2000). Nevertheless, this regulatory sequence does not conduct a high expression of genes of interest on its own, since it needs the fusion of the sequence of basal promoter of gene *RBCS2* (which is constitutive) to achieve a high expression.

Promoter *CYC6* reported by Ferrante *et al.* (2008) and described in patent US20110033938 (A1), is induced by the presence of nickel and the absence of copper in the medium, observing a disadvantage that the conducted expression of this promoter is low and the high concentrations of these metals cause toxicity over the growth of *C. reinhardtii,* in addition to the fact that NiCl₂ is a high cost inductor and its handling can be toxic to the human being.

Promoter LHCBM9 recently evaluated by Sawyer *et al.* (2016) is a promoter sequence induced by the deprivation of sulphates in the medium (TAP), with the disadvantage that required 48 of induction. The evaluation of this promoter is not conclusive in as much thus far its expression levels under induction have only been compared to the wild strain of *Chlamydomonas reinhardtii* and not with other promoters.

Promoter LIP (Park *et al.* 2013; Baek *et al.* 2016) is an inducible sequence by light (patent application US 2015/0147782 A1) and has shown a major expression compared to commercial promoter *HSP70A*/*RBCS2* but has shown the disadvantage that it needs a special regulation to change the intensity of light that requires to be inducible.

Promotor HSP90B was recently identified but its potential applications as an inducible promoter by the thermal shock is still being studied (Traewachiwiphak *et al.* 2018).

Nonetheless and due to the disadvantages each of these promoters show, none of them is found in the market yet, as a nuclear inducible promoter capable of conducting a high expression of recombinant proteins in *C. reinhardtii* that shows growth rates compelling for the industry.

On the other hand, the enzyme Glycerol-3-phosphate dehydrogenase (GPDH) is involved in the synthesis of glycerol and the metabolism of lipids, as shown in yeasts, and catalyzes the transformation of dihydroxyacetone phosphate (DHAP) and NADH (Nicotinamide and adenine dinucleotide in its reduced form) to glycerol-3-phosphate (G-3-P) and NAD+ (Nicotinamide and adenine dinucleotide in its oxidized form). Likewise, the G-3-P is used in the cell as a substrate to obtain glycerol, or be used as precursor to trigger the route of the triacylglycerides (TAGs) (Gancedo *et al.* 1968; Goshal *et al.* 2002; Vigeolas *et al.* 2007). Genes that codify for enzymes GPDH have been found in the salt water microalgae *Dunaliella salina* and *Dunalliela viridis* and the relation between the expression of these genes and the glycerol biosynthesis in these microalgae has been studied (He *et al.* 2007; 2009; Cai *et al.* 2013; Chen *et al.* 2011).

Regarding the fresh water green microalga *Chlamydomonas reinhardtii,* it is known that it possesses a defense mechanism against osmotic stress that consists in the accumulation and excretion of glycerol, and this response has been observed in the presence of osmotic stress by exposure to salts like NaCl and KCI (Husic and Tolbert 1986). In a previous study in our laboratory (Herrera-Valencia *et al.* 2012), three genes homologous to genes that codify for the enzyme GPDH were identified and characterized in *C. reinhardtii: CrGPDH1, CrGPDH2* and *CrGPDH3;* all these sequences showed a significant similarity with the *GPDH* genes of the microalga *Dunaliella salina* and *Dunaliella viridis.* By using a prediction program of signal peptide, it was found that only CrGPDH2 and CrGPDH3 proteins contain in its N-terminal end a signal peptide for the chloroplast, consequently they very possibly correspond to a GPDHs with location in this organelle. Regarding *CrGPDH1,* it is suggested that it codifies for a protein with cytosolic localization. By means of RT-PCR analysis it was observed that the expression of *CrGPDH2* and *CrGPDH3* was low, so for CrGPDH3 it was barely detectable by electrophoresis in an agarose gel, but that could be induced with a treatment of 200 mM of NaCl for two hours (Herrera-Valencia et al. 2012). Therefore an interest to investigate the potential of the promoter of *CrGPDH3* to conduct the expression of recombinant genes in an inducible way emerged. In a later study made in our laboratory (Casais-Molina *et al.* 2016), the functional characterization of *CrGPDH2* and *CrGPDH3* was made and it was demonstrated that these genes codify for enzymes with activity of GPDH and it was suggested that they are involved in osmoregulation since it was observed that the expression of these genes was induced in a treatment range that goes from 5 mM up to 200 mM of NaCl and an exposure time that goes from 5 up to 120 minutes. These results, suggested that *CrGPDH2* and *CrGPDH3* may be involved in the accumulation of glycerol and TAGs in response to conditions of osmotic stress and that these play an important role for the survival of *C. reinhardtii* under these conditions.

The advantages offered by having an inducible nuclear promoter that conducts a high protein expression of biotechnological interest in the green microalga *C. reinhardtii* have already been mentioned, furthermore, it is desirable that the inductor of this promoter be easily obtained, easy to apply, of economic price and that can be used in low concentrations to avoid affecting the cellular metabolism. It is desirable that this promoter may be used to direct the genetic expression in a controlled manner in green microalgae as the green microalga *C. reinhardtii* and other microalgae, even in other plant cells. Genes which expression could be directed by this promoter include those of importance in basic scientific research, to evaluate the function of genes in certain phenomena, metabolic pathways or phenotypes, as well as genes of biotechnological importance whose proteins have applications in human medicine or veterinary medicine such as vaccines, antibodies and other therapeutic and diagnostic proteins, but also other proteins used for human and animal feed, such as enzymes, or even proteins high in essential amino acids. In the state of the art the use of the viral constitutive promoter CaMV of the cauliflower mosaic virus is known, which has been successfully used in plant cells and microalgae (Kumar *et al.* 2004).

Based on these precedents, the study of the promoting region of gene *CrGPDH3* was made, obtaining the promoter described in this invention, which shows, in regard to the aforementioned promoters, the advantage of being a nuclear promoter, regulated with inductors that are easily obtained, applied, that are of low toxicity and of low cost such as the NaCl and others that are described in this invention. The promoter described herein includes both the core promoter as well as the region that grants the inducibility; in the same manner its capacity to conduct the expression of a recombinant protein in an inducible manner in photosynthetic organisms is also described, which for example, is significantly higher to the one reported for commercial promoter *HSP70A*/*RBCS2.*

### SUMMARY OF THE INVENTION

A first embodiment of the invention is to provide new nucleotide sequences and methods for the regulation of the genetic expression in photosynthetic organisms. The new nucleotide sequences correspond to the inducible promoter of the glycerol-3-phosphate dehydrogenase gene (*CrGPDH3*) isolated from the green microalga *Chlamydomonas reinhardtii* (promoter *CrGPDH3*).

Another embodiment of the invention is to provide functional nucleotide sequences comprised within the sequence of the inducible promoter of *CrGPDH3,* which are capable of directing the expression of a nucleotide sequence functionally joined to these sequences in host cells.

An additional embodiment of the invention is to provide a nucleotide sequence that corresponds to a core promoter, which has the capacity of directing the expression of a nucleotide sequence functionally joined to this sequence in host cells.

Even further, another embodiment of this invention consists in providing nucleotide sequences that correspond to the region responsible for the inducibility of the promoter *CrGPDH3* which can be functionally joined to the promoter concerned in this invention or to other promoters granting them the ability to direct the inducible expression of a nucleotide sequence functionally joined to these sequences in host cells.

Additionally, another embodiment of this invention consists in providing sequences that allow the elaborations of DNA constructs useful for the regulation of the genetic expression, that comprise any of the following sequences: a) for the sequence of the promoter *CrGPDH3,* b) by the sequence that correspond to the core promoter of *CrGPDH3,* c) by any of the nucleotide sequences that comprise the region that grants inducibility functionally joined to the core promoter of *CrGPDH3,* d) by any of the nucleotides sequences of the region that grants inducibility functionally joined to a heterologous promoter. All of them capable of directing the inducible expression of a heterologous gene in cells of photosynthetic organisms. The embodiments of the invention include expression vectors and photosynthetic cells that have incorporated in their genome any of these constructs in a stable manner.

Another embodiment consists in providing a method to express in a selective and controlled manner a nucleotide sequence in photosynthetic cells by means of the nuclear transformation of the cell with any of the DNA constructs describes in this invention and a heterologous nucleotide sequence of interest functionally joined to the pertaining construct (promoter), and on which such promoter initiates the transcription in an inducible manner of such nucleotide sequence in the cell by means of the use of an inductor. This method is useful for the expression of heterologous genes with basic research purposes or with biotechnological purposes like for example the recombinant protein production in host cells of photosynthetic organisms.

Furthermore, another embodiment of the invention is to provide the nucleotide sequences corresponding to the primers that allow the identification, isolation and cloning of promoter *CrGPDH3* and the functional sequences derived from it.

Lastly, another embodiment of this invention is to provide a method for the recombinant protein production in photosynthetic organisms, which production may be induced by means of easy control, low toxicity and low cost.

### BRIEF DESCRIPTION OF THE FIGURES

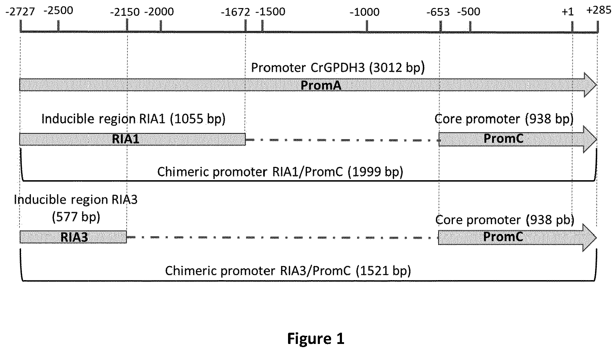

Figure 1 shows an outline of promoter *CrGPDH3* that corresponds to the fragment of 3012 bp named PromA, as well as the functional sequences that comprise it. These sequences include the core promoter PromC with a size of 938 bp; as well as the sequences corresponding to the inducible region RIA1 with a size of 1055 bp and RIA3 with a size of 577 bp. In the same way, the fusions that allow to obtain the chimeric promoters RIA1/PromC and RIA3/PromC are outlined. In the figure the location and size of the 5'UTR (+1 to +285) is indicated, as well as the location of the start codon of the transcription (+1).

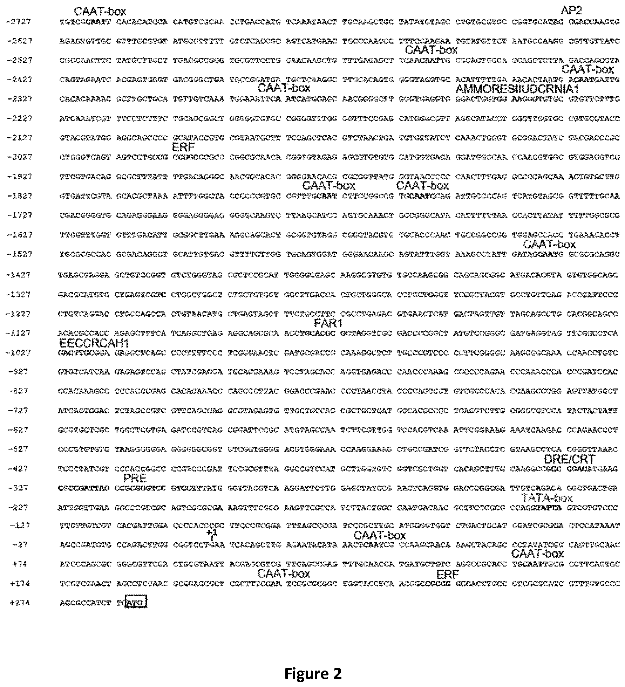

Figure 2 shows the sequence of promoter *CrGPDH3* and some putative *cis* elements, shown in bold, which were identified with the programs PlantCare and PlantPAN. The starting site of the transcription is indicated with +1 and the start codon of the translation (ATG) is enclosed in a frame. The TATA box (TATA-box) is an element belonging to the basal promoter in which the basal transcription levels are controlled. The CAAT box (CAAT-box) is an element commonly found in eukaryotic promoters and in enhancer regions. Other putative response elements are indicated with their corresponding names.

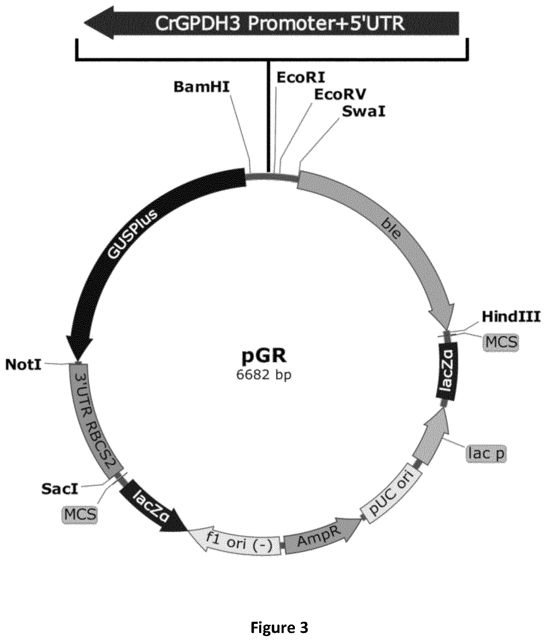

Figure 3 shows the map of the transformation vector for *C. reinhardtii* (pSP124S) that contains the expression construct in which different evaluated regions of promoter *CrGPDH3* were inserted. The inserted regions are indicated with an arrow corresponding to the Promoter *CrGPDH3* (*CrGPDH3* Promoter +5'UTR) or any of the nucleotide sequences comprised in the promoter or its fusions described in this invention. *GUSPlus* refers to the nucleotide sequence of the reporter gene that codifies for the glucuronidase enzyme, while 3'UTR RBCS2 refers to the 3' untranslated region of gene *RBCS2* of *C. reinhardtii.* ble means that the plasmid contains a resistance gene to spectinomycin that allows the selection of clones of *C. reinhardtii* that have been transformed. The restriction sites are indicated with the names of the enzymes they correspond to: *Eco*RI*, Bam*HI, *Not*I and *Sac*I.

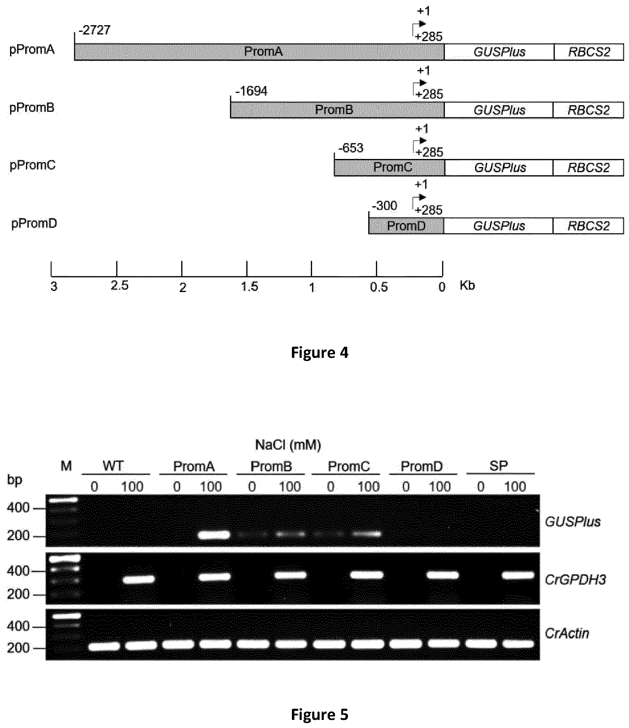

Figure 4 illustrates the progressive deletions made to promoter *CrGPDH3* with the purpose of elucidating the core promoter. The deletions are identified as PromA (3012 bp, +285 to -2727), PromB (1979 bp, +285 to -1694), PromC (938 bp, +285 to -653), and PromD (585 bp, +285 to -300). The arrow indicates the starting site of the 5' untranslated region (5'UTR, +285), followed by the reporter gene *GUSPlus* and terminator RBCS2.
Figure 5 shows the image of an agarose gel with the results of the RT-PCR evaluation of the expression of *GUSPlus* in *C. reinhardtii* conducted by different lengths of promoter *CrGPDH3*: PromA, PromB, PromC and PromD. The treatments with 0 and 100 mM of NaCl were applied at the 7^{th} day of cultivation for two hours. In the image the expected amplicon of 180 bp for *GUSPlus* is shown. WT = Wild Strain; SP= transformation vector pSP124S without the expression construct; M= Molecular weight marker. The expression of native gene *CrGPDH3* is also shown as a control that the treatments of NaCl were correct, and the expression of gene *CrActin* as a control that there was no contamination by genomic DNA on the evaluated samples.

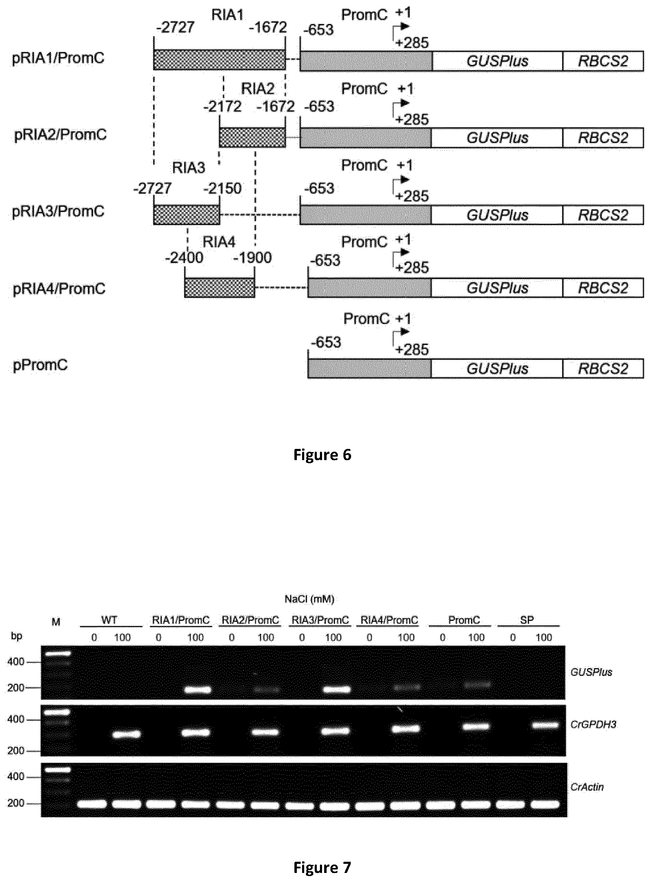

Figure 6 illustrates the fusions of different regions of promoter *CrGPDH3* with the core promoter (PromC). The fragments RIA1 (-2727 to -1672), RIA2 (-2172 to -1672), RIA3 (-2727 to -2150) and RIA4 (-2400 to -1900) were amplified using specific primers with the restriction site *Eco*RV in the 5' end and *Eco*RI in the 3' end. To obtain the different fusions to be evaluated, after the digestion with these enzymes, the products were fused to vector pSP124S upstream of core promoter *PromC,* followed by *GUSPlus* and RBCS2. Each expression construct was cloned into the transformation vector and each plasmid was named according to the contained construct, for example pRIA1/PromC.

Figure 7 shows an image of agarose gel with the results of the evaluation of the expression by RT-PCR of gene *GUSPlus* conducted by the different fusions of promoter *CrGPDH3* (RIA/PromC, RIA2/PromC, RIA3/promC, RIA4/PromC) in comparison with the core promoter *PromC.* The treatment with 0 and 100 mM of NaCl were applied at the 7^{th} day of cultivation for two hours. WT = Wild strain; SP= empty vector pSP124S; M= Molecular weight marker. The expression of native gene *CrGPDH3* is also shown as a control that the NaCl treatments were correct, and the expression of gene *CrActin* as a control that there was no contamination by genomic DNA on the evaluated samples.

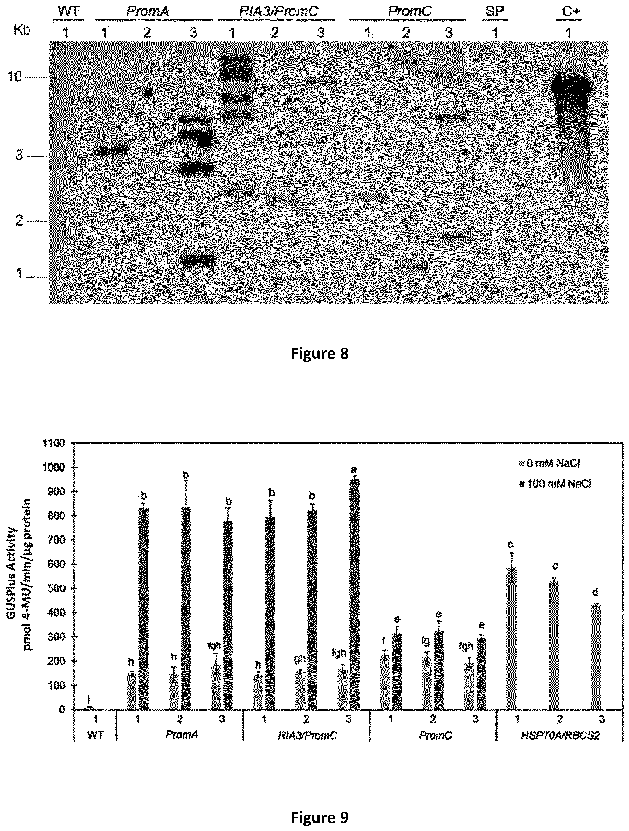

Figure 8 shows an image of a radiography with the result from the analysis by Southern blot of transgenic lines of *C. reinhardtii* transformed with expression constructs that contain gene *GUSPlus* being conducted either by promoter *PromA,* chimerical promoter *RIA3*/*PromC,* and core promoter *PromC.* A specific probe was used (fragment of marked sequence) to detect the *GUSPlus* gene. WT= Wild strain; SP= Empty vector pSP124S; C₊= DNA from plasmid of PromA (positive control). With this result it can be seen that there are three different transgenic lines: 1, 2 and 3 for each promoter to be evaluated (columns), and each line has a different number of copies of the inserted gene in its genome, which is seen as strips in each column, indicating at least one to five copies of the gene.
Figure 9 shows the result of the quantitative analysis of the activity of the glucuronidase enzyme (GUS) in three transgenic lines (1, 2 and 3) in *C. reinhardtii* transformed with different expression constructs that contain *GUSPlus* gene conducted by the evaluated sequences or fusions of promoter *CrGPDH3*: *PromA, RIA3*/*PromC* and *PromC. HSP70A*/*RBCS2* corresponds to 3 transgenic lines (1 to 3) of *C. reinhardtii* transformed with plasmids that carry expression construes that contain the *GUSPlus* gene conducted by the constitutive promoter *HSP70A*/*RBCS2.* The treatments with 0 and 100 mM of NaCl during two hours were applied at the 7^{th} day of cultivation and the measurement of the activity was made using a fluorimetric test. Each value is the average ± standard deviation (n=3). Different letters indicate significant differences among the averages (one way ANOVA, Fisher's LSD, P < 0.05).

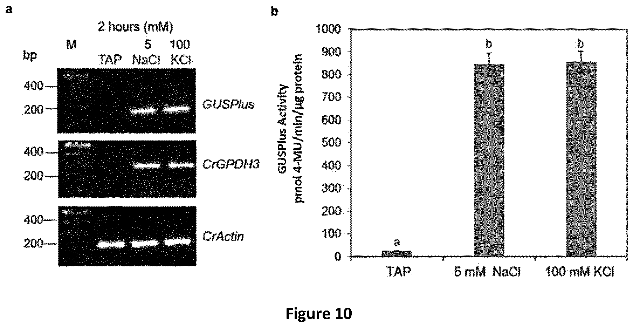

Figure 10 shows the inducibility results by different inductors using the chimeric promoter *RIA3*/*PromC* with different treatments at different concentrations. The treatments of 5 mM of NaCl and 100 mM of KCI were applied at the 7^{th} day of cultivation during two hours. A) Shows an agarose gel where the expression of the reporter gene *GUSPlus* is observed drived by *RIA3*/*PromC,* as well as the expression of native gene *CrGPDH3* as control that the treatments were correct and the constitutive gene *CrActin* as control that there was no contamination by genomic DNA on the evaluated samples. TAP= Without treatment; M= Molecular weight marker. B) Shows the result of the quantitative analysis of the activity of the glucuronidase enzyme (GUSPlus) when *GUSPlus* gene is conducted by *RIA3*/*PromC.* The measurement of the activity was by means of a fluorometric test. Each value is the average ± standard deviation (n=3). Different letters indicate significant differences among the averages (one way ANOVA, Fisher's LSD, P < 0.05). TAP= Without treatment.

### DETAILED DESCRIPTION OF THE INVENTION

This invention provides an inducible nuclear promoter isolated from the fresh water green microalga *Chlamydomonas reinhardtii* (promoter *CrGPDH3*), which is originally found conducting the expression of gene *CrGPDH3* that codes for the glycerol 3-phosphate dehydrogenase enzyme; this promoter represents an alternative for the regulation of the genetic expression in photosynthetic organisms, like for example the green microalga *C. reinhardtii.* The entire sequence of promoter *CrGPDH3* includes the sequence of the 5' untranslated region (5' UTR) of gene *CrGPDH3* and it is detailed in SEQ ID NO: 1. Furthermore, this invention includes nucleotide sequences that have at least 90% identity with the SEQ ID NO: 1 (*PromA*) and that have the capacity to direct the inducible expression of a functionally joined nucleotide sequence. In the same way it includes a core promoter *(PromC,* SEQ. ID. NO: 2) and an inducible region that is found both in the RIA1 sequence (SEQ. ID NO: 3), as well as in RIA3 sequence (SEQ. ID. NO: 4), with which constructs can be generated that maintain their functionality when any of these sequences is functionally joined to a promoter, as it happens with the SEQ. ID. NO. *5* (*RIA1*/*PromC*) and with SEQ. ID. NO: 6 (*RIA3*/*PromC*). Thus, this invention includes nucleotide sequences that have at least 90% identity with the SEQ ID NO:2, and with the SEQ ID NO: 4.

### Attainment of sequences for the regulation of the genetic expression

The identification and isolation of the sequence of promoter region of gene *CrGPDH3* was made *in silico* based on the generated data about the cloning of gene *CrGPDH3* (Herrera-Valencia *et al.* 2012; Casais-Molina *et al.* 2016) as described in example 1.

Subsequently, an expression vector was prepared which contained the reporter gene *GUSPlus,* as described in example 2, on which all the fragments that were a product of the deletion analysis of promoter *CrCPGDH3* were cloned, and which helped as transformation vector for the transformation by biolistic of the microalga *C. reinhardtii,* as described in example 3. Once cultures of *C. reinhardtii* putatively transformed were obtained, the corresponding analysis of the expression of reporter gene *GUSPlus* were made by RT-PCR as described in example 4.

Having the putative promoter sequence of gene *CrGPDH3* identified, it was amplified on the basis the genomic DNA of *C. reinhardtii* using specific oligonucleotide primers (designed particularly for this purpose), whose sequences are described in table 1 and correspond to the SEQ. ID. NO: 7 and SEQ. ID. NO: 8. Once this was amplified it was cloned in a plasmid and it was sequenced to verify its integrity as described in example 5.

The isolation, amplification, cloning in multiplication vector, sequencing and cloning in a transformation vector of the sequences pertaining to promoter *CrGPDH3,* core promoter and inducibility regions was made using the method that comprises the following steps:
a) Extraction of nucleic acid from *C. reinhardtii.*
b) PCR amplification using specific oligonucleotide primers according to Table 1.
c) Insertion of the amplicon in a vector by a ligation enzyme and multiplication of the resulting plasmid in bacterial cultures.
d) Extraction and purification of the plasmid.
e) Sequencing and confirmation of the integrity of the sequence.
f) Cloning of the sequence in an adequate transformation plasmid.

**Table 1. Sequences from the specific primers used to amplify the sequences pertaining to this invention.**

| Name of the sequence to be amplified | Sequence of the oligonucleotide (5' → 3') | Position of the primer |
|---|---|---|
| Promoter *CrGPDH3* (*PromA*) | TAAGATATCTGTCGCAATTCACACATCCAC | -2727 |
| | SEQ. ID. NO: 7 | |
| | TGGGGATCCGAAGATGGCGCTGGGCACAAAC | +285 |
| | SEQ. ID. NO: 8 | |
| Core promoter (*PromC*) | AGCGAATTCGTCTTGCGGGCGTCCATACTAC | -653 |
| | SEQ. ID. NO: 9 | |
| | TGGGGATCCGAAGATGGCGCTGGGCACAAAC | +285 |
| | SEQ. ID. NO: 10 | |
| Inducibility region (RIA1) | TAAGATATCTGTCGCAATTCACACATCCAC | -2727 |
| | SEQ. ID. NO: 11 | |
| | TACGAATTCGCACTGGATGCTTAAGACTTGC | -1672 |
| | SEQ. ID. NO: 12 | |
| Inducibility region (RIA3) | TAAGATATCTGTCGCAATTCACACATCCAC | -2727 |
| | SEQ. ID. NO: 13 | |
| | CTAGAATTCTATGCCTAACGCCCATGCTCGG | -2150 |
| | SEQ. ID. NO: 14 | |

In examples 5 and 7 the attainment of the expression construct is described in pGR for the deletion analysis that allow the identification both of the core promoter as well as for the inducibility region by means of RT-PCR.

Promoter *CrGPDH3* (*PromA,* SEQ. ID NO: 1) comprises a core promoter identified as SEQ. ID NO: 2, and a region that grants inducibility to the promoter, comprised in any of the sequences identified as SEQ ID NO: 3 or SEQ. ID. NO: 4 (Figure 1). This invention includes a fusion of SEQ. ID NO: 2 with SEQ. ID NO: 3 or SEQ. ID NO: 4, which are capable of driving the inducible expression of a nucleotide sequence that has been functionally joined to these in a host cell and that in this document are identified as SEQ. ID. NO: 5 and SEQ. ID. NO: 6, respectively.

SEQ. ID NO: 2 corresponding to the core promoter can be used individually since it has the capacity to direct the expression of a functionally joined nucleotide sequence, in a constitutive manner, as described in example 6. This core promoter can be used to conduct the expression of genes of interest in photosynthetic organisms like *C. reinhardtii,* with various purposes which include, but are not limited to, to modulate the genetic expression to observe a specific phenotype, alter a metabolic pathway, create a recombinant protein of interest in scientific research or of importance in the biotechnology industry.

Any of the SEQ ID NO: 3 and SEQ. ID. NO: 4, corresponding to the inducibility region, can be used functionally joined to a promoter, this being either the core promoter of this invention (SEQ. ID NO: 2) (example 8) or to any other heterologous promoter to grant it inducibility and increase its expression levels with various purposes which include, but are not limited to, modulate the genetic expression to observe a certain phenotype, alter a metabolic pathway, create a recombinant protein of interest in scientific research or of importance in the biotechnology industry.

SEQ ID NO: 5 and SEQ. ID. NO: 6, corresponding to the core promoter (SEQ ID NO: 2) fused respectively to the inducibility region SEQ ID NO: 3 and SEQ ID NO: 4 can be used to direct the expression of a functionally joined nucleotide sequence, to grant it inducibility and with high expression levels, with various purposes that include but are not limited to modulate the genetic expression to observe a certain phenotype, alter a metabolic pathway, create a recombinant protein of interest in scientific research or of importance in the biotechnology industry. It is worth noting that the SEQ ID NO: 5 (1999 bp) and SEQ ID NO: 6 (1521 bp) were fused to the corresponding sequences using an enzyme restriction strategy, in this case using the sequence of the restriction site corresponding to the *Eco*RI enzyme (it could have been any other adequate restriction enzyme), consequently each fusion contains inside the GAATTC sequence in the binding site of the chimeric promoter.

Each one of the sequences and constructs described earlier were identified and designed by means of a cloning strategy of the sequences corresponding to promoter *CrGPDH3,* using restriction enzymes. As a first step, the core promoter was identified as described in examples 5 and 6, in a second stage the inducibility regions were identified, which were tested developing constructs with the core promoter (examples 7 and 8).

This invention includes those different forms or fusions to attain chimeric promoters that include any of the sequences described herein. Furthermore, the performance of promoter *CrGPDH3* (*PromA*), the chimeric promoter (*RIA3*/*PromC*), and the core promoter (*PromC*) were compared with regard to the commercial constitutive promoter *HSP70A*/*RBCS2* by means of a quantitative test of the activity of the reporter protein GUSPlus (examples 9 and 10), proving that *PromA* and *RIA3*/*PromC* show a better performance than promoter *HSP70A*/*RBCS2,* a widely used commercial promoter, which supports the use of the promoters described in this invention for the recombinant protein production with basic research purposes as well as their use in the industry of recombinant protein production of socioeconomic interest.

Finally, the chimeric promoter *RIA3*/*PromC* is capable of conducting the expression of reporter gene GUSPlus in a similar way than native promoter under conditions of 5 mM of NaCl or 100 mM of KCI, and when the activity of protein GUSPlus is evaluated from these same samples, it is observed that the levels are similar to those when 100 mM of NaCl are used (example 11). These facts indicate the versatility of the promoter and the economic advantage that its use can represent, since a treatment of low-cost as salt is used at a concentration of barely 5 mM for an adequate and desirable performance.

Once a strain of *C. reinhardtii,* or any other photosynthetic organism, is found transformed with one of the constructs that contain the promoter of *CrGPDH3* or any of the sequences corresponding to this invention, this strain can be used to produce, extract and purify the recombinant protein of interest. In the case of a transformed strain of *C. reinhardtii* it can be maintain with subcultures every two months in Petri dishes in medium solid TAP at 25 ± 2°C in a photoperiod cycle of 16/8 h light/darkness, or on vials with medium solid TAP at 16°C with subcultures every up to six months. To obtain the recombinant protein of interests, a small portion of the culture is transferred (for example: one colony) of the strain of interests from the solid medium at 50 mL of medium liquid TAP in a 200 mL flask. Once the culture reaches the end of the exponential growth phase, which can be past seven days in the basal cultivation condition, at 25 ± 2°C in a photoperiod cycle of 16/8 h light/darkness, the inductor is added, which can be an inorganic salt, preferably at a concentration lower than 200 mM, to quote an example, NaCl can be used in a concentration range (as an example but not limited to) which can go from 5 to 200 mM, or else using KCI at a concentration, as an example but not limited to, of a 100 mM as described in example 11. After two hours of cultivation in these conditions the activity of the protein of interest can already be detected, and therefore its accumulation in the aforementioned strain. From this culture, it is possible to extract and purify the protein of interest in accordance to protocols well known in the literature. It can also be chosen to do the escalation of the cultivation, for such, once the culture has reached the end of the exponential growth phase, which can be past seven days in the basal cultivation conditions, at 25 ± 2 ºC in a photoperiod cycle of 16/8 h light/darkness, such will inoculate at a volume each time greater, and depending on the container or bioreactor, or cultivation method, it can come to be, for example but not limited to, of up to 100 000 L.

The general process to carry out the recombinant protein production in a system that uses the promoter or the sequences subject of this invention consists on the stages of:
a) Introducing any of the sequences or constructs described in this invention in an adequate organism for the expression of recombinant proteins.
b) Cultivate the organism in the optimum conditions for its proliferation.
c) Induce the protein production by means of the addition of an inductor, which is selected from the inorganic salts like NaCl and KCI, on concentrations lower than 200 mM.
d) Extract the protein from the culture medium.
e) Purify the protein.

### EXAMPLES

**Example 1.** Selection and isolation of a putative sequence from the promoter region of *CrGPDH3* gene.

In order to select the putative sequence of the promoter region of *CrGPDH3* gene, a region of 3012 bp upstream the start codon of the transcription (ATG) was chosen from *CrGPDH3* as putative promoter region, which includes a sequence of 2727 bp upstream from the transcription starting site (+1) and the 5' untranslated region (5' UTR) (+285 bp) of *CrGPDH3* (Casais-Molina et al. 2016). This sequence was named PromA (Fig. 1) which contains 3012 bp and corresponds to the SEQ ID NO. 1. PromA was amplified by PCR using the oligonucleotide primers described in table 1, by means of which the restriction sites *Eco*RI and *Bam*HI were added to their ends, they were cloned in the pGEM-T Easy vector and the integrity of the cloned sequences was confirmed via sequencing. All the sequences were edited using the software Lasergene version 7.2 (DNA-STAR). The sequence PromA was analyzed using the database programs PLANTCARE and PlantPLAN for the identification of putative *cis* response elements (Fig.2). The PromA sequence isolated was used for the deletion analyses that were subsequently made.

**Example 2.** Preparation of the vector for cloning the expression constructs.

To prepare the vector that was used to insert each expression construct and transform the microalgae, the following procedure was followed. First, the coding sequence of the reporter gene *GUSPlus*™ (*GUSPlus*) was amplified by PCR from pCAMBIA 1305.1 (Cambia) using specific oligonucleotides, named GusplusORF F [5' ACTGGATCCAACAATGGTAGATCTGAGGGTAAATTTC 3'] and GusplusORF R [5' TGCGCGGCCGCTCACACGTGATGGTGATGGTGATG 3'], by means of which the restriction sites *Eco*RI and *Bam*HI were added to their ends, they were cloned in vector pGEM-T Easy and the integrity of the cloned sequences was confirmed by sequencing. Subsequently, this sequence was cloned in pSP124S (Lumbreras et al. 1998) obtained from the Chlamydomonas Resource Center, and now this new plasmid was named pG. The 3' untranslated region (3' UTR) of gene RBCS2 was used as terminator and it was obtained from pHdp70A/RbcS2-cEpo (Heitzer and Zschoernig 2007, Chlamydomonas Resource Center) by means of a strategy of restriction enzymes using the enzymes *Not*I and *Sac*I and linked upstream of *GUSPlus* in pG, this way generating the expression vector named pGR (Fig. 3).

**Example 3.** Transformation of *C. reinhardtii* through biolistic.

The nuclear transformation of *C. reinhardtii* was made using biolistic (microparticle bombardment). The cells of *C. reinhardtii* were cultivated in 50 mL of TAP until reaching the end of the exponential stage, they were collected by centrifugation and 4 x 10⁷ cells were plated in Petri dishes with 25 mL of medium solid TAP. The reagents and microparticles were prepared as it has been described by Daniell *et al.* (2005) using 10 µg of plasmid DNA and 0.5 µg of gold microparticles of 0.6 µg (Bio-Rad). For the bombardment a PDS-1000/He biolistic chamber (Bio-Rad) was used, using the parameters for algae and the specific adjustments from the supplier. After bombardment, the cells were stored in darkness for 24 h, after which they were collected and transferred to fresh solid TAP medium supplemented with 25 µg of zeocin (Invitrogen, USA) as selection agent. The colonies resistant to zeocin, and therefore putatively transformed, began to appear after 7 to 10 days.

**Example 4.** Expression analysis of the reporter gene *GUSPlus* by RT-PCR.

In order to identify the core promoter, and the inducibility region of promoter *CrGPDH3,* a strategy was made that included the analysis of the expression of reporter gene *GUSPlus* by RT-PCR. The experiments were made the following way: nine putatively transformed colonies resistant to zeocin originated from the particle bombardment were randomly selected; each colony was cultivated separately in 3 mL of TAP medium for seven days, after which approximately 50 µg from each culture were transferred separately to solid TAP medium and they were stored for subsequent uses. The rest of the cultures were mixed and homogenized in one single Erlenmeyer flask and then were divided en equal amounts in two flasks. Each flask received a different treatment. For example, one flask received a treatment of 100 mM of NaCl for 2 h while the other received distilled water as control.

For the analysis that allow to determine the induction of promoter *CrGPDH3* with different inductors, the experiments were made in the following way: 10,000 cells/mL were inoculated per flask and were cultivated until the end of the exponential phase, then one flask received a treatment of 5 mM of NaCl and other flask a treatment of 100 mM of KCI for 2 h, while yet another flask received distilled water as a control. The cells were collected by centrifugation, frozen with liquid nitrogen and stored at - 80º C until their use. The total RNA was extracted as described by Herrera-Valencia et al. (2012). The expression of *GUSPlus* was evaluated by RT-PCR. The first cDNA strand was synthetized using 8 µg of total RNA and 200 U of SuperScript II reverse transcriptase (Invitrogen, USA) following the instruction of the manufacturer. For the PCR analysis 200 µM of each dNTP were used, 0.2 µM of each oligonucleotide primer (GusPlus 159 F [5' ACGTGGAGATTCCGAATGTC 3'] and GusPlus159 R [5' TGTTGATGAGGAACTTGCCG 3'], 5 µL of each cDNA dilution (1:5), 1.5 µM of MgCl₂, 1 X of PCR buffer and 1 U of *Taq* DNA polymerase (Invitrogen) in a final volume of 50 mL.

The PCR was made with the following cycling conditions: 95 °C for 3 min, and then 40 cycles of 95 °C for 30 s, 68 °C for 60 s, and 72 °C for 1 min, in a C1000 Thermal Cycler (BioRad). The expression of the native *CrGPDH3* gene was used as a positive control for the NaCl treatment, as described by Herrera-Valencia *et al.* (2012). Native constitutive gene *CrActin* was used as a control to indicate that similar amounts of RNA was used as templates and that there was no contamination by genomic DNA, as described by Herrera-Valencia (2012).

**Example 5.** Attainment of the expression constructs in pGR for the deletion analysis that allowed the confirmation of the functionality of *CrGPDH3* promoter and the identification of the core promoter by RT-PCR.

In order to identify the region that contains the core promoter and evaluate its inducibility, serial deletions drawn from PromA were generated (SEQ ID NO. 1), these being named PromB (- 1964 to +285), PromC (-653 to +285) and PromD (-300 to +285) (Fig. 4). All the fragments were amplified by PCR using specific oligonucleotides for each one (Table 2), integrating to them the restriction sites *Eco*RV and *Eco*RI in the 5' and 3' ends, respectively. The products of the amplification were cloned to vector p-GEM-T-Easy (Promega) and the identity of the sequences was verified by sequencing (Macrogene Corp.). Subsequently, each fragment was cloned in the expression vector pGR, upstream the reporter gene *GUSPlus,* thus obtaining plasmids with the respective expression constructs (Fig. 4).

**Table 2. Sequences of the oligonucleotide primers that were used for the deletion analysis that allowed for the confirmation of the functionality of CrGPDH3 promoter and the identification of the core promoter by RT-PCR.**

| Name | Sequence (5' to 3') | Restriction site |
|---|---|---|
| PromA F | TAAGATATCTGTCGCAATTCACACATCCAC | *Eco*RI |
| PromA R | TGGGGATCCGAAGATGGCGCTGGGCACAAAC | *Bam*HI |
| PromB F | AATGAATTCGCAAGTCTTAAGCATCCAGTGC | *Eco*RI |
| PromB R | TGGGGATCCGAAGATGGCGCTGGGCACAAAC | *Bam*HI |
| PromC F | AGCGAATTCGTCTTGCGGGCGTCCATACTAC | *Eco*RI |
| PromC R | TGGGGATCCGAAGATGGCGCTGGGCACAAAC | *Bam*HI |
| PromD F | TTCGAATTCATGGGTTACGTCAAGGATTC | *Eco*RI |
| PromD R | TGGGGATCCGAAGATGGCGCTGGGCACAAAC | *Bam*HI |

**Example 6.** Deletion analysis for the confirmation of the functionality of the *CrGPDH3* promoter and the identification of the core promoter by RT-PCR.

In order to evaluate which of the fragments described in example 5 (Fig. 4) are capable of driving the expression of reporter gene *GUSPlus* in an inducible way, various cultures were obtained by microparticle bombardment with the plasmids that contained the respective expression constructs described in example 5 and that contained the different fragments of promoter *CrGPDH3*: PromA, PromB, PromC, PromD (Fig. 4). After microparticle bombardment, nine putatively transformed colonies resistant to zeocin were randomly selected for each promoter fragment, and the analysis that allow for the identification of the core promoter was made, as described in example 4. As it can be seen in Figure 5, the result of this analysis allowed us to determine that the sequence we selected as putative promoter of *CrGPDH3,* PromA, was capable of driving the expression of *GUSPlus* reporter gene in a similar way than the native promoter, under treatment of 100 mM of NaCl, while no amplicon was observed in the treatment without NaCl. On the other hand, the fragment of PromB did not render a positive result. These results also allowed us to identify PromC as the core promoter of *CrGPDH3,* since it was the shortest fragment on which a constitutive expression of *GUSPlus* was still observed, although a much lower level of expression was qualitatively observed compared to PromA, as well as a slight induction in the treatment of 100 mM of NaCl. On the other hand, PromD did not render a positive result (Fig. 5). Finally, all of these results indicated that the inducibility region must be found upstream of PromB, in a region between -2727 and -1694 (Fig. 4).

**Example 7.** Attainment of the expression constructs in pGR for the deletion analysis that allowed the identification of the inducibility region by RT-PCR.

In order to map the region that contains the elements that grant the inducibility to promoter *CrGPDH3,* a variety of fragments were amplified drawn from the sequence of PromA: RIA1 (-2727 to -1672), RIA2 (-2172 to -1672), RIA3 (-2727 to -2150) and RIA4 (-2400 to -1900) (Fig. 6). All the fragments were amplified by PCR using specific oligonucleotides for each one (Table 3), integrating to them the restriction sites *Eco*RV and *Eco*RI in the 5' and 3' ends, respectively. Finally, the products of the amplification were cloned to vector p-GEM-T-Easy (Promega) and the identity of the sequences was verified by sequencing (Macrogene Corp.). Subsequently, each fragment was cloned in pPromC (plasmid that contains the sequence PromC), upstream from the core promoter *PromC,* thus obtaining the expression constructs for the deletion analysis that allowed for the identification of the inducibility region of the promoter. The constructs were then named RIA1/PromC, RIA2/PromC, RIA3/PromC and RIA4/PromC.

Table 3. Sequences of the oligonucleotide primers that were used for the deletion analysis that allowed for the identification of the inducibility region of *CrGPDH3* promoter by RT-PCR.

| Name | Sequence (5' to 3') | Restrictio n site |
|---|---|---|
| RIA1 F | TAAGATATCTGTCGCAATTCACACATCCAC | *Eco*RV |
| RIA1 R | TACGAATTCGCACTGGATGCTTAAGACTTGC | *Eco*RI |
| RIA2 F | TATGATATCGAATCACAGCTTGAGAATAC | *Eco*RV |
| RIA2 R | TACGAATTCGCACTGGATGCTTAAGACTTGC | *Eco*RI |
| RIA3 F | TAAGATATCTGTCGCAATTCACACATCCAC | *Eco*RV |
| RIA3 R | CTAGAATTCTATGCCTAACGCCCATGCTCGG | *Eco*RI |
| RIA4 F | TAAGATATCTGATGCCGGATGATGCTCAAGGC | *Eco*RV |
| RIA4 R | CTTGAATTCCTGTCAAAATAAAGCGCCTGTCA | *Eco*RI |

**Example 8.** Deletion analysis for the identification of the inducibility region of the promoter by RT-PCR.

In order to evaluate which of the fragments described in example 7 (Fig. 6) was capable of driving the expression of the *GUSPlus* reporter gene in an inducible way, various cultures were obtained by micropartcile bombardment with the plasmids that contained the respective expression constructs described in example 7 and that contained the different fusions of the fragments of PromA with *PromC:* RIA1/PromC, RIA2/PromC, RIA3/PromC and RIA4/PromC (Fig. 6). After microparticle bombardment, nine putatively transformed colonies resistant to zeocin were randomly selected, and the analysis that allow for the identification of the inducibility region was carried out, following the protocol described in example 4. As it can be seen in Figure 7, the result of this analysis allowed us to determine that the fusions *RIA1*/*PromC* and *RIA3*/*PromC* were capable of driving the expression of the *GUSPlus* reporter gene in a similar way than the native promoter, under treatment of 100 mM of NaCl, while no amplicon (band) was observed in the treatment without NaCl. These results allowed to identify the inducibility region of the promoter *CrGPDH3* in the region of 577 bp of RIA3 between -2727 and - 1672 (Fig. 6), region also included in RIA1 and, thus, also in any deletion between RIA1 and RIA3.

**Example 9.** Analysis by Southern blot to identify transgenic lines of *C. reinhardtii.*

In order to identify transgenic lines that contain each one of the constructs with the promoters that turned out to be successful, three bombarded lines were analyzed that contained either *PromA, RIA3*/*PromC* or *PromC.* A bombarded line was included with empty vector pGR (without expression construct) marked as SP. As positive control, purified pPromA was used (plasmid that contains promoter sequence *PromA*), marked as C+. The total genomic DNA was extracted from 1 x 10⁵ of cells of *C. reinhardtii* using the Illustra™ Nucleon Phytopure DNA Extraction system (GE Healthcare). Approximately 15 µg of DNA were used to digest with the restriction enzymes *Pst*I and *Bam*HI all night at 37 °C, they were separated in an agarose gel at 1% using as marker 2-Log DNA marker (New England Biolabs) and subsequently transferred to a nylon membrane as described in Sambrook and Russell (2001). A probe of 159 bp was prepared through amplification by PCR from *GUSPlus* gene marked with digoxigenin using a marking and detection DIG DNA kit (Roche). The hybridization was carried out according to the specifications of the manufacturer. At the end, as shown in Figure 8, three different transgenic lines were able to be identified (1 to 3) for each promoter to be evaluated, with different copies of the constructs inserted in their genome, with at least one copy, up to at least five copies (bands observed in each lane). As expected, in the negative control (SP) there is no band observed, while in the positive control (C+) just one band is observed with the expected weight.

**Example 10.** Quantitative assay of GUSPlus activity.

The activity of the GUSPlus reporter protein, allowed us to determine indirectly the performance of each promoter that was evaluated in this study. Thus, in order to evaluate the activity of GUSPlus, a quantitative assay was carried out as described by Jefferson *et al.* (1987). Cells of *C. reinhardtii* were cultivated for 7 days (until the end of the exponential growth phase), and subsequently the corresponding treatment was applied. In one assay three biological replicas of each transgenic line were evaluated, in each biological replica the effect of a different treatment was evaluated. For example, a flask received the treatment of 100 mM of NaCl for 2 h while the other received distilled water as control. In another assay, three biological replicas of each transgenic line were also evaluated, in each biological replica the effect of a different treatment was evaluated, but in this case a flask received 5 mM of NaCl and other 100 mM of KCI for 2 h, while another flask received distilled water as control. Approximately 100 mg of fresh weight were collected from each sample and frozen with liquid nitrogen. The frozen samples were mixed with the extraction buffer of GUS for the fluorometric tests of GUS (50 mM of phosphate buffer at pH 7.0, 10 mM EDTA, 0.1% Triton X-100, 0.1% SDS and 10 mM β-mercaptoethanol and were lysed for 1 min using a digital polytron Ultra Turrax® T25 (IKA). Subsequently, the samples were centrifuged at 13,000 g for 20 min at 4 °C. The supernatant was used to measure the concentration of proteins using a commercial kit, the Quick Start Brandford Protein Assay Kit 1 (BioRad) with albumin serum as standard. 15 µg of protein extract were used which were mixed with the buffer from the assay which contained 1 mM of 4-Methylumbelliferyl-b-glucunoride (4-MUG) (Sigma-Aldrich) at 37°C. The reaction was finished with the addition of 200 mM Na₂CO₃ to a final concentration of 180 mM. The activity of GUSPlus was measured determining the fluorescence in a Fluorometer QuantiFluor™ Handheld (Promega), with an excitation wavelength of 265 nm and an emission wavelength of 455 nm. The standard curve was created using 4-methylumbelliferone (4-MU) (Sigma-Aldrich). The activity of GUSPlus was expressed in picomoles of 4.MU per microgram of protein per minute.

The activity analysis of GUSPlus was carried out by fluorimetry in three transgenic lines (each one with three biological replicas) that contained as promoter in its expression construct whether *PromA, RIA3*/*PromC* or *PromC.* As it can be seen in figure 9, both in the samples of *PromA* and of *RIA3*/*PromC* a significantly higher enzymatic activity could be seen (approximately four times higher) when they were exposed to 100 mM of NaCl than when they were not, although a basal enzymatic activity of GUSPlus is observed with these promoters.

In figure 9 the basal levels of activity can also be appreciated in the samples of *PromC,* in which a small increase can be quantitatively observed, but statistically significant when the samples were exposed to 100 mM of NaCl compared to when they were exposed to 0 mM of NaCl. A remarkable result of this analysis was that the activity of GUSPlus was significantly higher (around 40%) for the transgenic lines that contained whether the promoter *PromA* or the chimeric promoter *RIA3*/*PromC* in treatment of 100 mM of NaCl when compared with three transgenic lines (each one with three biological replicas) that contained as promoter in its expression constructs the commercial constitutive promoter *HSP70A*/*RBCS2* in basal conditions. As expected, there was no detectable activity of GUSPlus in the sample of the wild strain (negative control). This analysis allowed us to verify the usefulness of the varieties of *CrGPDH3* promoter for the recombinant protein production, whether *PromA* functionally joined to a gene, or the regions that grant inducibility joined to either *PromC* or any other promoter to make it inducible and increase its expression levels, and that can also be translated in a high enzymatic activity.

**Example 11.** Induction of promoter *CrGPDH3* (*RIA3*/*PromC*) with different inductors.

In order to investigate the versatility of *CrGPDH3* promoter as far as the inductors that can be used to initiate the expression of the gene of interest, *RIA3*/*PromC* chimeric promoter was chosen to evaluate its performance in treatments of 5 mM of NaCl (to which the native promoter was induced as mentioned in Casais-Molina *et al.* 2016) and 100 mM of KCI, which has been reported as inductor of the glycerol synthesis in which gene *CrGPDH3* is involved (Casais-Molina *et al.* 2016; Husic and Tolbert 1986). As it can be observed in figure 10a, the chimeric promoter *RIA3*/*PromC* was capable of inducing the expression of *GUSPlus* both at 5 mM of NaCl and at 100 mM of KCI in a similar way to native promoter *CrGPDH3.* In the same way, the activity of GUSPlus both for the treatment of 5 mM of NaCl as of 100 mM of KCI was similar between both treatments (figure 10b) and it presented itself in a similar way than when 100 mM of NaCl were used in example 10. This result indicates that for the induction of the expression using the varieties of promoter *CrGPDH3* of this invention a range of, for example (but not limited to) 5 to 200 mM of NaCl could be used, as well as other salts like for example, but not limited to, KCI in similar ranges.

## Claims

1. An inducible promoter useful for the expression of recombinant proteins when it is functionally joined to a heterologous gene to express, wherein said inducible promoter is a nuclear promoter obtained from the green microalga *Chlamydomonas reinhardtii* and its sequence comprises 90% of identity with SEQ. ID. NO: 1.

2. The inducible promoter according to claim 1, wherein said promoter is inducible through a treatment with an inorganic salt, where the inorganic salt is selected from group: NaCl and KCI.

3. A core promoter useful for the expression of recombinant proteins, wherein said core promoter is obtained from the inducible promoter according to claim 1 and possesses a sequence which comprises 90% identity with SEQ. ID. NO: 2.

4. An inducible region, useful for the induction of the expression of recombinant proteins, wherein said inducible region is obtained from the inducible promoter according to claim 1 and its sequence comprises 90% identity with any of the sequences SEQ. ID. NO: 3 or SEQ. ID. NO: 4.

5. The inducible region according to claim 4, wherein said inducible region is activated through a treatment with an inorganic salt, where the inorganic salt is selected from group: NaCl and KCI.

6. An inducible chimeric promoter useful for the expression of recombinant proteins when it is functionally joined with a heterologous gene to express, wherein the inducible chimeric promoter comprising the core promoter according to claim 3.

7. An inducible chimeric promoter useful for the expression of recombinant proteins when it is functionally joined to a heterologous gene to express, wherein said inducible chimeric promoter comprises the inducible region according to claim 4.

8. An inducible chimeric promoter useful for the expression of recombinant proteins when it is functionally joined to a heterologous gene to express, wherein said inducible chimeric promoter comprises a sequence that corresponds to 90% of identity with any of the sequences SEQ. ID. NO: 5 or SEQ. ID. NO: 6.

9. A pair of forward and reverse primers, useful for the amplification of the inducible promoter according to claim 1, wherein said forward and revers primers comprising the sequences SEQ. ID. NO: 7 and SEQ. ID. NO: 8, respectively.

10. A pair of forward and reverse primers, useful for the amplification of the core promoter according to claim 3, wherein said forward and reverse primers comprising the sequences SEQ. ID. NO: 9 and SEQ. ID. NO: 10, respectively.

11. A pair of forward and reverse primers, useful for the amplification of the inducible region according to claim 4, wherein said forward and reverse primers comprising the sequences SEQ. ID. NO: 11 and SEQ. ID. NO: 12, respectively.

12. A pair of forward and reverse primers, useful for the amplification of the inducible region according to claim 4, wherein said forward and reverse primers comprising the sequences SEQ. ID. NO: 13 and SEQ. ID. NO: 14, respectively.

13. A method to induce the expression of recombinant proteins that includes the steps of:
a) introducing an expression construct that includes the sequence of the inducible promoter in an adequate organism for the expression of recombinant proteins;
b) cultivate the organism in optimum conditions for its proliferation;
c) induce the production of proteins through the addition of an inductor;
d) extract the protein from the culture medium; and
e) purify the protein;
wherein the sequence of said inducible promoter includes any of the sequences selected from group: SEQ. ID. NO: 1, SEQ. ID. NO: 2, SEQ. ID. NO: 3, SEQ. ID. NO: 4, SEQ. ID. NO: 5 and SEQ. ID. NO: 6.

14. The method to induce the expression of recombinant proteins according to claim 13, wherein the inductor is an inorganic salt, where the inorganic salt is chosen from group: NaCl and KCI.

15. The method to induce the expression of recombinant proteins according to claim 14, wherein the inductor in the culture medium is applied at a concentration lower than 200 mM.


SEQUENCE LISTING

<110> CENTRO DE INVESTIGACIÓN CIENTÍFICA DE YUCATÁN, A.C.
<120> PROMOTOR INDUCIBLE DEL GEN CrGPDH3 DE Chlamydomonas reinhardtii Y SU
USO PARA LA EXPRESIÓN DE PROTEÍNAS RECOMBINANTES
<160> 14

<210> 1
<211> 3012
<212> ADN
<213> Chlamydomonas reinhardtii
<400> 1
tgtcgcaatt cacacatcca catgtcgcaa cctgaccatg tcaaataact tgcaagctgc 60
tatatgtagc ctgtgcgtgc cggtgcatac cgaccaagtg agagtgttgc gtttgcgtgt 120
atgcgttttt gtctcaccgc agtcatgaac tgcccaaccc tttccaagaa tgtatgttct 180
aatgccaagg cgttgttatg cgccaacttc tatgcttgct tgaggccggg tgcgttcctg 240
gaacaagctg tttgagagct tcaacaattg cgcactggca gcaggtctta gaccagcgta 300
cagtagaatc acgagtgggt gacgggctga tgccggatga tgctcaaggc ttgcacagtg 360
gggtaggtgc acatttttga aacactaatg acaatgattg cacacaaaac gcttgctgca 420
tgttgtcaaa tggaaattca atcatggagc aacggggctt gggtgaggtg ggactggtgg 480
aagggtgtgc gtgttctttg atcaaatcgt ttcctctttc tgcagcggct gggggtgtgc 540
cggggtttgg ggtttccgag catgggcgtt aggcatacct gggttggtgc cgtgcgtacc 600
gtacgtatgg aggcagcccc gcataccgtg cgtaatgctt tccagctcac gtctaactga 660
tgtgttatct caaactgggt gcggactatc tacgacccgc ctgggtcagt agtcctggcg 720
ccggcccgcc cggcgcaaca cggtgtagag agcgtgtgtg catggtgaca ggatgggcaa 780
gcaaggtggc gtggaggtcg ttcgtgacag gcgctttatt ttgacagggc aacggcacac 840
ggggaacacg cgcggttatg ggtaaccccc caactttgag gccccagcaa agtgtgcttg 900
gtgattcgta gcacgctaaa attttggcta ccccccgtgc cgtttgcaat cttccggccg 960
tgcaatccag attgccccag tcatgtagcg gtttttgcaa cgacggggtg cagagggaag 1020
gggaggggag ggggcaagtc ttaagcatcc agtgcaaact gccgggcata cattttttaa 1080
ccacttatat ttttggcgcg ttggtttggt gtttgacatt gcggcttgaa aggcagcact 1140
gcggtgtagg cgggtacgtg tgcacccaac tgccggccgg tggagccacc tgaaacacct 1200
tgcgcgccac gcgacaggct gcattgtgac gttttcttgg tgcagtggat gggaacaagc 1260
agtatttggt aaagcctatt gatagcaatg gcgcgcaggc tgagcgagga gctgtccggt 1320
gtctgggtag cgctccgcat tggggcgagc aaggcgtgtg tgccaagcgg cagcagcggc 1380
atgacacgta gtgtggcagc gacgcatgtg ctgagtcgtc ctggctggct ctgctgtggt 1440
ggcttgacca ctgctgggca cctgctgggt tcggctacgt gcctgttcag accgattccg 1500
ctgtcaggac ctgccagcca ctgtaacatg ctgagtagct ttctgccttc cgcctgagac 1560
gtgaactcat gactagttgt tagcagcctg cacggcagcc acacgccacc agagctttca 1620
tcaggctgag aggcagcgca acctgcacgc gctaggtcgc gaccccggct atgtccgggc 1680
gatgaggtag ttcggcctca gacttgcgga gaggctcagc cccttttccc tcgggaactc 1740
gatgcgaccg caaaggctct tgcccgtccc ccttcggggc aaggggcaaa ccaacctgtc 1800
gtgtcatcaa gagagtccag ctatcgagga tgcaggaaag tcctagcacc aggtgagacc 1860
caacccaaag cgccccagaa cccaaaccca cccgatccac ccacaaagcc cccacccgag 1920
cacacaaacc cagcccttac ggacccgaac ccctaaccta ccccagccct gtcgcccaca 1980
ccaagcccgg agttatggct atgagtggac tctagccgtc gttcagccag gcgtagagtg 2040
ttgctgccag cgctgctgat ggcacgccgc tgaggtcttg cgggcgtcca tactactatt 2100
gcgtgctcgc tggctcgtga gatccgtcag cggattccgc atgtagccaa tcttcgttgg 2160
tccacgtcaa attcggaaag aaatcaagac ccagaaccct cccgtgtgtg taagggggga 2220
ggggggcggt gtcggtgggg acgtgggaaa ccaaggaaag ctgccgatcg gttctacctc 2280
gtaagcctca cgggttaaac tccctatcgt cccaccggcc ccgtcccgat tccgcgttta 2340
ggccgtccat gcttggtgtc ggtcgctggt cacagctttg caaggccggc cgacatgaag 2400
cgccgattag ccgcgggtcc gtcgtttatg ggttacgtca aggattcttg gagctatgcg 2460
aactgaggtg gacccggcga ttgtcagaca ggctgactga attggttgaa ggcccgtcgc 2520
agtcgcgcga aagtttcggg aagttcgcca tcttactggc gaatgacaac gcttccggcg 2580
ccaggtatta gtcgtgtccc ttgttgtcgt cacgattgga ccccacccgc ttcccgcgga 2640
tttagcccga tcccgcttgc atggggtggt ctgactgcat ggatcgcgga ctccataaat 2700
agccgatgtg ccagacttgg cggtcctgaa tcacagcttg agaatacata aactcaatcg 2760
ccaagcaaca aagctacagc cctatatcgg cagttgcaac atcccagcgc gggggttcga 2820
ctgcgtaatt acgagcgtcg ttgagccgag tttgcaacca tgatgctgtc aggccgcacc 2880
tgcaattgcg ccttcagtgc tcgtcgaact agcctccaac gcggagcgct cgctttccaa 2940
tcggcgcggc tggtacctca acggccgccg gccacttgcc gtcgcgcatc gtttgtgccc 3000
agcgccatct tc 3012

<210> 2
<211> 938
<212> ADN
<213> Chlamydomonas reinhardtii
<400> 2
gtcttgcggg cgtccatact actattgcgt gctcgctggc tcgtgagatc cgtcagcgga 60
ttccgcatgt agccaatctt cgttggtcca cgtcaaattc ggaaagaaat caagacccag 120
aaccctcccg tgtgtgtaag gggggagggg ggcggtgtcg gtggggacgt gggaaaccaa 180
ggaaagctgc cgatcggttc tacctcgtaa gcctcacggg ttaaactccc tatcgtccca 240
ccggccccgt cccgattccg cgtttaggcc gtccatgctt ggtgtcggtc gctggtcaca 300
gctttgcaag gccggccgac atgaagcgcc gattagccgc gggtccgtcg tttatgggtt 360
acgtcaagga ttcttggagc tatgcgaact gaggtggacc cggcgattgt cagacaggct 420
gactgaattg gttgaaggcc cgtcgcagtc gcgcgaaagt ttcgggaagt tcgccatctt 480
actggcgaat gacaacgctt ccggcgccag gtattagtcg tgtcccttgt tgtcgtcacg 540
attggacccc acccgcttcc cgcggattta gcccgatccc gcttgcatgg ggtggtctga 600
ctgcatggat cgcggactcc ataaatagcc gatgtgccag acttggcggt cctgaatcac 660
agcttgagaa tacataaact caatcgccaa gcaacaaagc tacagcccta tatcggcagt 720
tgcaacatcc cagcgcgggg gttcgactgc gtaattacga gcgtcgttga gccgagtttg 780
caaccatgat gctgtcaggc cgcacctgca attgcgcctt cagtgctcgt cgaactagcc 840
tccaacgcgg agcgctcgct ttccaatcgg cgcggctggt acctcaacgg ccgccggcca 900
cttgccgtcg cgcatcgttt gtgcccagcg ccatcttc 938

<210> 3
<211> 1055
<212> ADN
<213> Chlamydomonas reinhardtii
<400> 3
tgtcgcaatt cacacatcca catgtcgcaa cctgaccatg tcaaataact tgcaagctgc 60
tatatgtagc ctgtgcgtgc cggtgcatac cgaccaagtg agagtgttgc gtttgcgtgt 120
atgcgttttt gtctcaccgc agtcatgaac tgcccaaccc tttccaagaa tgtatgttct 180
aatgccaagg cgttgttatg cgccaacttc tatgcttgct tgaggccggg tgcgttcctg 240
gaacaagctg tttgagagct tcaacaattg cgcactggca gcaggtctta gaccagcgta 300
cagtagaatc acgagtgggt gacgggctga tgccggatga tgctcaaggc ttgcacagtg 360
gggtaggtgc acatttttga aacactaatg acaatgattg cacacaaaac gcttgctgca 420
tgttgtcaaa tggaaattca atcatggagc aacggggctt gggtgaggtg ggactggtgg 480
aagggtgtgc gtgttctttg atcaaatcgt ttcctctttc tgcagcggct gggggtgtgc 540
cggggtttgg ggtttccgag catgggcgtt aggcatacct gggttggtgc cgtgcgtacc 600
gtacgtatgg aggcagcccc gcataccgtg cgtaatgctt tccagctcac gtctaactga 660
tgtgttatct caaactgggt gcggactatc tacgacccgc ctgggtcagt agtcctggcg 720
ccggcccgcc cggcgcaaca cggtgtagag agcgtgtgtg catggtgaca ggatgggcaa 780
gcaaggtggc gtggaggtcg ttcgtgacag gcgctttatt ttgacagggc aacggcacac 840
ggggaacacg cgcggttatg ggtaaccccc caactttgag gccccagcaa agtgtgcttg 900
gtgattcgta gcacgctaaa attttggcta ccccccgtgc cgtttgcaat cttccggccg 960
tgcaatccag attgccccag tcatgtagcg gtttttgcaa cgacggggtg cagagggaag 1020
gggaggggag ggggcaagtc ttaagcatcc agtgc 1055

<210> 4
<211> 577
<212> ADN
<213> Chlamydomonas reinhardtii
<400> 4
tgtcgcaatt cacacatcca catgtcgcaa cctgaccatg tcaaataact tgcaagctgc 60
tatatgtagc ctgtgcgtgc cggtgcatac cgaccaagtg agagtgttgc gtttgcgtgt 120
atgcgttttt gtctcaccgc agtcatgaac tgcccaaccc tttccaagaa tgtatgttct 180
aatgccaagg cgttgttatg cgccaacttc tatgcttgct tgaggccggg tgcgttcctg 240
gaacaagctg tttgagagct tcaacaattg cgcactggca gcaggtctta gaccagcgta 300
cagtagaatc acgagtgggt gacgggctga tgccggatga tgctcaaggc ttgcacagtg 360
gggtaggtgc acatttttga aacactaatg acaatgattg cacacaaaac gcttgctgca 420
tgttgtcaaa tggaaattca atcatggagc aacggggctt gggtgaggtg ggactggtgg 480
aagggtgtgc gtgttctttg atcaaatcgt ttcctctttc tgcagcggct gggggtgtgc 540
cggggtttgg ggtttccgag catgggcgtt aggcata 577

<210> 5
<211> 1999
<212> ADN
<213> Chlamydomonas reinhardtii
<400> 5
tgtcgcaatt cacacatcca catgtcgcaa cctgaccatg tcaaataact tgcaagctgc 60
tatatgtagc ctgtgcgtgc cggtgcatac cgaccaagtg agagtgttgc gtttgcgtgt 120
atgcgttttt gtctcaccgc agtcatgaac tgcccaaccc tttccaagaa tgtatgttct 180
aatgccaagg cgttgttatg cgccaacttc tatgcttgct tgaggccggg tgcgttcctg 240
gaacaagctg tttgagagct tcaacaattg cgcactggca gcaggtctta gaccagcgta 300
cagtagaatc acgagtgggt gacgggctga tgccggatga tgctcaaggc ttgcacagtg 360
gggtaggtgc acatttttga aacactaatg acaatgattg cacacaaaac gcttgctgca 420
tgttgtcaaa tggaaattca atcatggagc aacggggctt gggtgaggtg ggactggtgg 480
aagggtgtgc gtgttctttg atcaaatcgt ttcctctttc tgcagcggct gggggtgtgc 540
cggggtttgg ggtttccgag catgggcgtt aggcatacct gggttggtgc cgtgcgtacc 600
gtacgtatgg aggcagcccc gcataccgtg cgtaatgctt tccagctcac gtctaactga 660
tgtgttatct caaactgggt gcggactatc tacgacccgc ctgggtcagt agtcctggcg 720
ccggcccgcc cggcgcaaca cggtgtagag agcgtgtgtg catggtgaca ggatgggcaa 780
gcaaggtggc gtggaggtcg ttcgtgacag gcgctttatt ttgacagggc aacggcacac 840
ggggaacacg cgcggttatg ggtaaccccc caactttgag gccccagcaa agtgtgcttg 900
gtgattcgta gcacgctaaa attttggcta ccccccgtgc cgtttgcaat cttccggccg 960
tgcaatccag attgccccag tcatgtagcg gtttttgcaa cgacggggtg cagagggaag 1020
gggaggggag ggggcaagtc ttaagcatcc agtgcgaatt cgtcttgcgg gcgtccatac 1080
tactattgcg tgctcgctgg ctcgtgagat ccgtcagcgg attccgcatg tagccaatct 1140
tcgttggtcc acgtcaaatt cggaaagaaa tcaagaccca gaaccctccc gtgtgtgtaa 1200
ggggggaggg gggcggtgtc ggtggggacg tgggaaacca aggaaagctg ccgatcggtt 1260
ctacctcgta agcctcacgg gttaaactcc ctatcgtccc accggccccg tcccgattcc 1320
gcgtttaggc cgtccatgct tggtgtcggt cgctggtcac agctttgcaa ggccggccga 1380
catgaagcgc cgattagccg cgggtccgtc gtttatgggt tacgtcaagg attcttggag 1440
ctatgcgaac tgaggtggac ccggcgattg tcagacaggc tgactgaatt ggttgaaggc 1500
ccgtcgcagt cgcgcgaaag tttcgggaag ttcgccatct tactggcgaa tgacaacgct 1560
tccggcgcca ggtattagtc gtgtcccttg ttgtcgtcac gattggaccc cacccgcttc 1620
ccgcggattt agcccgatcc cgcttgcatg gggtggtctg actgcatgga tcgcggactc 1680
cataaatagc cgatgtgcca gacttggcgg tcctgaatca cagcttgaga atacataaac 1740
tcaatcgcca agcaacaaag ctacagccct atatcggcag ttgcaacatc ccagcgcggg 1800
ggttcgactg cgtaattacg agcgtcgttg agccgagttt gcaaccatga tgctgtcagg 1860
ccgcacctgc aattgcgcct tcagtgctcg tcgaactagc ctccaacgcg gagcgctcgc 1920
tttccaatcg gcgcggctgg tacctcaacg gccgccggcc acttgccgtc gcgcatcgtt 1980
tgtgcccagc gccatcttc 1999

<210> 6
<211> 1521
<212> ADN
<213> Chlamydomonas reinhardtii
<400> 6
tgtcgcaatt cacacatcca catgtcgcaa cctgaccatg tcaaataact tgcaagctgc 60
tatatgtagc ctgtgcgtgc cggtgcatac cgaccaagtg agagtgttgc gtttgcgtgt 120
atgcgttttt gtctcaccgc agtcatgaac tgcccaaccc tttccaagaa tgtatgttct 180
aatgccaagg cgttgttatg cgccaacttc tatgcttgct tgaggccggg tgcgttcctg 240
gaacaagctg tttgagagct tcaacaattg cgcactggca gcaggtctta gaccagcgta 300
cagtagaatc acgagtgggt gacgggctga tgccggatga tgctcaaggc ttgcacagtg 360
gggtaggtgc acatttttga aacactaatg acaatgattg cacacaaaac gcttgctgca 420
tgttgtcaaa tggaaattca atcatggagc aacggggctt gggtgaggtg ggactggtgg 480
aagggtgtgc gtgttctttg atcaaatcgt ttcctctttc tgcagcggct gggggtgtgc 540
cggggtttgg ggtttccgag catgggcgtt aggcatagaa ttcgtcttgc gggcgtccat 600
actactattg cgtgctcgct ggctcgtgag atccgtcagc ggattccgca tgtagccaat 660
cttcgttggt ccacgtcaaa ttcggaaaga aatcaagacc cagaaccctc ccgtgtgtgt 720
aaggggggag gggggcggtg tcggtgggga cgtgggaaac caaggaaagc tgccgatcgg 780
ttctacctcg taagcctcac gggttaaact ccctatcgtc ccaccggccc cgtcccgatt 840
ccgcgtttag gccgtccatg cttggtgtcg gtcgctggtc acagctttgc aaggccggcc 900
gacatgaagc gccgattagc cgcgggtccg tcgtttatgg gttacgtcaa ggattcttgg 960
agctatgcga actgaggtgg acccggcgat tgtcagacag gctgactgaa ttggttgaag 1020
gcccgtcgca gtcgcgcgaa agtttcggga agttcgccat cttactggcg aatgacaacg 1080

cttccggcgc caggtattag tcgtgtccct tgttgtcgtc acgattggac cccacccgct 1140
tcccgcggat ttagcccgat cccgcttgca tggggtggtc tgactgcatg gatcgcggac 1200
tccataaata gccgatgtgc cagacttggc ggtcctgaat cacagcttga gaatacataa 1260
actcaatcgc caagcaacaa agctacagcc ctatatcggc agttgcaaca tcccagcgcg 1320
ggggttcgac tgcgtaatta cgagcgtcgt tgagccgagt ttgcaaccat gatgctgtca 1380
ggccgcacct gcaattgcgc cttcagtgct cgtcgaacta gcctccaacg cggagcgctc 1440
gctttccaat cggcgcggct ggtacctcaa cggccgccgg ccacttgccg tcgcgcatcg 1500
tttgtgccca gcgccatctt c 1521

<210> 7
<211> 30
<212> ADN
<213> Chlamydomonas reinhardtii
<400> 7
taagatatct gtcgcaattc acacatccac 30

<210> 8
<211> 31
<212> ADN
<213> Chlamydomonas reinhardtii
<400> 8
tggggatccg aagatggcgc tgggcacaaa c 31

<210> 9
<211> 31
<212> ADN
<213> Chlamydomonas reinhardtii
<400> 9
agcgaattcg tcttgcgggc gtccatacta c 31

<210> 10
<211> 31
<212> ADN
<213> Chlamydomonas reinhardtii
<400> 10
tggggatccg aagatggcgc tgggcacaaa c 31

<210> 11
<211> 30
<212> ADN
<213> Chlamydomonas reinhardtii
<400> 11
taagatatct gtcgcaattc acacatccac 30

<210> 12
<211> 31
<212> ADN
<213> Chlamydomonas reinhardtii
<400> 12
tacgaattcg cactggatgc ttaagacttg c 31

<210> 13
<211> 30
<212> ADN
<213> Chlamydomonas reinhardtii
<400> 13
taagatatct gtcgcaattc acacatccac 30

<210> 14
<211> 31
<212> ADN
<213> Chlamydomonas reinhardtii

<400> 14
ctagaattct atgcctaacg cccatgctcg g 31